# EUROPEAN PATENT SPECIFICATION

(11) **EP 4 238 609 B1**
(45) Date of publication and mention of the grant of the patent: **29.10.2025**
(21) Application number: 22159913.7
(22) Date of filing: 03.03.2022
(51) Int. Cl.: A61N 5/10

(54) **RADIOTHERAPY TREATMENT PLANNING FOR ION ARCS**
STRAHLENTHERAPIEBEHANDLUNGSPLANUNG FÜR IONENBÖGEN
PLANIFICATION DE TRAITEMENT DE RADIOTHÉRAPIE POUR ARCS IONIQUES

(43) Date of publication of application: 06.09.2023
(73) Proprietor: RaySearch Laboratories AB, 104 30 Stockholm (SE)
(72) Inventor: ENGWALL, Erik, 129 44 Hägersten (SE); BOKRANTZ, Rasmus, 182 47 Enebyberg (SE); MARTHIN, Otte, 723 58 Västerås (SE); WASE, Viktor, 126 31 Hägersten (SE); GLIMELIUS, Lars, 114 18 Stockholm (SE); FREDRIKSSON, Albin, 118 69 Stockholm (SE); ANDERSSON, Björn, 754 37 Uppsala (SE)

(56) References cited:
- EP-B1- 3 549 636
- WO-A1-2011/042819
- US-A1- 2017 213 690
- US-A1- 2018 085 596
- US-A1- 2020 101 323
- US-A1- 2021 260 406

## Description

### Technical Field

The present invention relates to the planning of ion-based radiotherapy treatment and in particular planning of treatment involving ion arcs.

### Background

In particle-based radiotherapy treatment, patients are irradiated with charged particles such as protons, helium or carbon ions. The particles are controlled in such a way that they will deposit most of their energy at specific depths in the patient so that the whole target will be covered. The depth of the Bragg peak in the patient can be controlled by adjusting the kinetic energy of the particles. The lateral position of the Bragg peak can be controlled using electromagnets to deflect the focused beam. This allows for the delivery of highly localized doses at well-controlled positions in the patient. A certain combination of kinetic energy and lateral deflection of the beam is referred to as a spot. The relative number of particles delivered to a spot is commonly referred to as the spot weight. The use of spots in many different locations in a three-dimensional space, enables fully covering the target volume with the desired dose distribution. This procedure is called active scanning ion beam therapy, also known as pencil beam scanning.

The kinetic energies of the spots are often, but not necessarily, distributed over a number of discrete energies. A group of spots with the same kinetic energy, but different lateral deflection is often referred to as an energy layer. All spots within one energy layer thus have the same energy level. To cover a desired volume in a patient, different energy layers, with different energy levels, are defined such that particles of a particular energy layer will deposit their energy at a certain (water equivalent) depth in the patient. The energy layers are selected in such a way that the Bragg peaks will be distributed over the volume to be treated.

The spot weights of each energy layer are determined in the treatment planning system through optimization, and the spot weights are iteratively varied to achieve desired objectives or constraints related to dose levels or other relevant quantities. The case where the spot weights are allowed to vary freely over the beams is referred to as intensity modulated ion therapy. In the common case of protons, it is referred to as intensity-modulated proton therapy (IMPT).

Ion arc therapy involves radiation in a sequence of a large number of principal irradiation directions, for example 10 or more, 50 or more, or 100 or more irradiation directions, each having a number of energy layers, for example up to 20 energy layers. The adjustment between each energy layer, and between each irradiation direction, takes time. Typically, only one energy layer is delivered per direction. The arc can be delivered either through a continuous moving arc where the radiation is delivered during movement, or controlled by a series of static beams describing a trajectory with stops where the beam is turned on. The arc is defined by control points, each pair of control points defining an initial and final energy level and a start and stop angle for the irradiation. Two adjacent control points may vary in irradiation angle, energy level or both. The start and stop angles are typically close to each other to ensure that the irradiation is delivered approximately from the principal irradiation direction. The control points are selected to achieve a short delivery time, while still maintaining a high-quality plan which will ensure effective treatment of the patient.

US2021/260406A1 refers to creating an ion-based radiotherapy treatment plan including a spot-weight optimization procedure arranged to create the plan involving an arc.

### Summary of the invention

The invention is defined in the appended claims. The present disclosure aims to provide ion arc treatment plans that will allow time-efficient delivery to a patient while ensuring a suitable dose distribution.

Hence, an aspect of the disclosure relates to a computer-implemented method of creating an ion-based radiotherapy treatment plan for treating a patient, the method including a spot-weight optimization procedure arranged to create the plan so that it involves an arc with a first part which is a first sub-arc, said sub-arc being a continuous sub-arc defined by a first set of control points defining a first set of energy levels distributed over a first trajectory involving different irradiation angles shifting the beam in a first direction, and so that the plan further involves a second part involving a second set of control points different from the first set of control points, the second set of control points defining a second set of energy levels, each of the first and second set including a plurality of energy levels, wherein
- the second part is a static beam and the second set of control points define several energy levels from one irradiation angle, or
- the second part is a second sub-arc and the second set of control points define a second trajectory along a second direction which is different from the first direction, or
- the second part involves a second sub-arc moving in the first direction and the relative movement between the gantry and the patient over the arc describes more than a full revolution around the patient, or
- the second part involves a second sub-arc at least partially overlapping the first sub-arc, and
wherein the method includes selecting the first and second sets of energy levels together.

By arranging the radiation in an arc having a first and a second part, where at least the first part is a sub-arc, a suitable dose distribution can be ensured, while the plan may be setup such that the delivery time is reduced by a suitable arrangement of the energy layers. The energy levels are selected together for the whole plan, so that they can complement each other across the treatment area. This enables a grouping of energy levels into the first and second sets in a way that will minimize the time needed for energy level changes during delivery. The first and second parts may be delivered in any suitable order, that is, the second part may be delivered before the first part. Where the first and the second part involve sub-arcs moving in the same direction, at least one of the first or the second sub-arc may describe a full revolution and the other sub-arc may describe a full or a partial revolution around the patient. Alternatively, both sub-arcs may describe partial revolutions which together describe more than a full revolution.

In some embodiments, the second part involves a static beam containing at least two different energy levels irradiated from the same direction. The static beam enables a higher number of energy levels delivered from one irradiation angle, compared to the energy layers included in the sub-arc, where typically one energy level is used for each principal irradiation direction.

In other embodiments, the second part involves a second sub-arc in a different direction from the first sub-arc, and the second set of energy levels is distributed over a second trajectory. The direction of the second sub-arc may be selected freely. In some cases, the direction may be opposite of the first direction. Having the beam turned on during gantry movement in both directions will save time in cases where the gantry is moving back and forth. Selecting the first and second directions independently will be feasible, for example, where translation and rotation are combined, for optimal coverage of the target.

In yet other embodiments, the second part involves a second sub-arc, at least partly overlapping the first sub-arc trajectory, the second direction being the same as the first direction. This is feasible, for example, if the relative movement between the gantry and the patient describes a full revolution around the patient. In such cases, each of the first and the second sub-arc may describe a full revolution or part of a revolution. In addition, in an embodiment where the irradiation angles are changed using a gantry, the patient may be moved during the treatment in a direction different from the movement of the irradiation device. This can be achieved by moving the support on which the patient is positioned. This may involve raising or lowering the chair, or by moving the couch horizontally. In this case the first and/or the second sub-arc will describe a spiral rather than a circle around the patient. Overlapping sub-arcs in the same direction may also be achieved by moving the gantry back and forth so it can move in the same direction more than once.

If the second sub-arc and the first sub-arc have overlapping portions, either in the same or in opposite directions, the irradiation angles of the sub-arcs may be the same, or may be selected to complement each other. For example, the irradiation angles of the second sub-arc can be shifted in order to achieve different principal irradiation directions in the second sub-arc than in the first sub-arc. Additionally, the energy levels in the second sub-arc may be mirrored with respect to the first sub-arc. This means that an irradiation angle that involves a high energy level for the first sub-arc will have a low energy level in the second arc, and vice versa.

As will be understood, the method is not limited to only a first and a second part; any number of parts of the same or different types may be combined. For example, a first sub-arc may be combined with several second parts of different types, for example, a first sub-arc followed by a static beam and a second sub-arc, or a static beam followed by a first sub-arc, followed by another static beam, and/or a second sub-arc.

The method may include grouping the energy layers of each part into at least a first and a second sector wherein the energy levels within each sector are arranged in order to reduce delivery time. This typically involves grouping the energy layers into sectors in such a way that the energy levels decrease within each sector, to reduce the number of increases of energy levels in the sub-arc, since increases in many systems take longer time than decreases. In the case of mirroring, the sectors from the first arc may be used for the second arc as well, and the energy levels be mirrored within each sector.

The selection of energy layers is made for all sub-arcs and static beams simultaneously. In some embodiments, the step of selecting the first and second, and possibly further, sets of energy layers together includes a pre-selection step, wherein the energy layers are selected prior to the spot-weight optimization procedure. The pre-selection of energy layers can be performed in order to use the same sectors in the first and second sub-arc. The sectors in the first and second sub-arcs can also be shifted with respect to each other in order to achieve large flexibility in the optimization while maintaining a short delivery time. The pre-selection may be performed by treating the first and second sub-arc as one single arc. Alternatively, the energy level selection is performed as part of a spot-weight optimization procedure based on dose-based optimization objectives.

Pre-selection could be based on any suitable criterion, for example, one of the following
- Maximizing covered target volume
- Minimizing overlap of covered target volume
- Maximizing goodness measures of covered target volume.

The methods according to the present disclosure may be combined with further techniques for improving the resulting plan. For example, robust optimization may be applied. A particularly favorable addition would be applying an energy level reduction technique on the 1st and 2nd set of energy levels together to reduce the total number of energy levels further. Examples of energy level reduction techniques are discussed, for example, in EP3549636A1 by the same applicant.

Aspects of the disclosure also relate to a computer program product comprising computer-readable code means which, when executed in a computer, will cause the computer to perform the method according to any one of the preceding claims. The computer program product may be stored in any suitable way but is preferably stored on a non-transitory storage medium. Aspects of the disclosure also relate to a computer having a processor and a program memory, wherein the program memory includes such a computer program product, stored in such a way that it can be executed in the processor.

The methods according to the disclosure enable the creation of time-efficient treatment plans by minimizing the need for time-consuming energy layer changes without sacrificing the flexibility needed to ensure a suitable dose distribution. Such changes, typically when changing to a higher energy, are known to be time-consuming, to the degree that scanning the patient over several revolutions, using one energy layer per irradiation direction may be faster than changing energy layers multiple times from the same or close directions. The methods will also allow to be combined with robust optimization so that the generated plans will be robust to uncertainties in, e.g., patient setup, patient anatomy and interpretation of density in different voxels of the patient. This is an important feature of any particle treatment plan.

### Brief description of drawings

The invention will be described in more detail in the following, by way of examples and with reference to the appended drawings.
Figs. 1a - 1d illustrate different possible combinations of sub-arcs and static beams according to embodiments of the invention.
Fig. 2 illustrates the selection of energy levels in two sub-arcs that are planned to constitute one arc according to one embodiment.
Fig. 3 is a flow chart illustrating some embodiments of an ion arc treatment planning method
Fig. 4 shows a computer system in which the method according to the invention may be implemented.

### Detailed description of embodiments

The present invention is suitable for planning any type of ion-based radiotherapy arc plans. In some examples below, protons are mentioned by way of example but it should be understood that the method is not limited to protons.

An arc may be defined as a beam that defines a trajectory on a patient. As explained above, the arc is typically defined by a sequence of control points, each consecutive pair of which defines a start and stop angle and an initial and a final energy layer. To form an arc, at least two control points must define different irradiation directions, to define different irradiation angles. In other words, each possible subset of three adjacent control points in the set of control points always has at least two consecutive control points that have different angles of irradiation relative to the patient. This means that the beam will enter the patient in at least two different points from at least two different directions because of relative movement between the beam and the patient. The movement may describe a rotation, which may be a full or partial revolution, or a translation or a combination of the two. A conventional arc rotates or moves in a single direction relative to the patient and as a full revolution or less.

An arc may be continuous in the sense that the beam is on during movement and thus between control points. The control points can be grouped in pairs. A pair of control points defines a gantry angle window over which the irradiation is delivered. In other words, if the current and the next control point are contained in the same control point pair, the irradiation is on between the control points. If the current and the next control point belong to different control point pairs, the irradiation may be turned off between these control points to provide the prescribed radiation to the patient before moving on to the next control point pair. Control points belonging to the same pair typically have the same energy level and the energy level is typically changed between control point pairs. It is desirable to reduce the number of energy layers for the arc, since both irradiation of an energy layer and changing between energy layers, and thus energy levels, takes time. For many machines, the time for switching the energy levels in one direction is substantially higher than switching in the other direction, e.g., changing from a high energy to a low energy is faster than switching from a low to a high energy. As an alternative or in addition to energy layer reduction, the sequence of energy layers may be selected in such a way that the changes and total delivery take as little time as possible.

The arc is achieved by a relative movement between the beam and the patient. This may be achieved by rotating the gantry, and/or influencing the direction of the beam by some other means such as magnets. It may also be achieved by moving the patient relative to the beam, for example by positioning the patient on a moving support such as a rotating chair or couch. Such moving patient supports are known in the art.

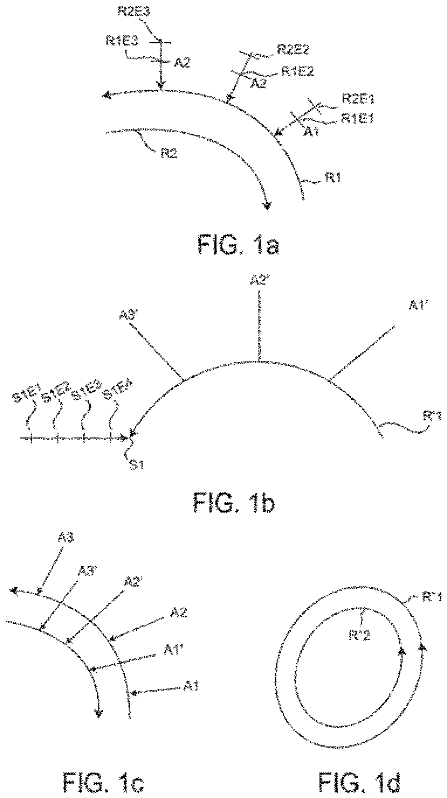

Figs. 1a, 1b, 1c, 1d illustrate different combinations of sub-arcs and/or static beams that may be implemented according to embodiments of the present invention. In each of the examples, the arc may be created by a relative movement between the gantry and the patient, which may be created by rotating the gantry, or by moving the patient support with the patient on it, or both. Rotating patient supports in the form of chairs or couches are known in the art. It is also possible to move the beam relative to the patient by other means, for example using magnets.

Fig. 1a shows an example in which the first and second parts of the arc are both sub-arcs, R1, R2 covering the same part of the patient's circumference, with opposite trajectories. As can be seen, the first sub-arc uses pairs of control points centered around three different irradiation angles A1, A2, A3, using energy layers R1E1, R1E2, R1E3 for angles A1, A2, A3 respectively. The second sub-arc R2 in this example uses the same irradiation angles A1, A2, A3 but different energy layers, R2E1, R2E2, R2E3 for angles A1, A2, A3. As will be understood, the sub-arcs could also follow the same trajectory in the same direction, if a rotating gantry is used it will usually save time to create the second sub-arc during a return movement instead of returning the gantry before delivering the second sub-arc.

Fig. 1b shows a different example in which the first and second parts of the arc are both sub-arcs, R1, R2 covering the same part of the patient's circumference, with opposite trajectories, or optionally with trajectories in the same direction. As can be seen, the first sub-arc uses three different irradiation angles A1, A2, A3, using energy layers R1E1, R1E2, R1E3 for angles A1, A2, A3 respectively. The second sub-arc R2 in this example uses different irradiation angles A1', A2', A3', and energy layers R2E1, R2E2, R2E3.

Fig. 1c shows an example in which the first part is a sub-arc R1' covering a part of the patient's circumference and the second part is a static beam S1. The sub-arc R1' involves a number of control points defining irradiation angles in the same way as in Fig. 1a. The static beam involves a number of energy layers S1E1, S1E2, S1E3, S1E4 being delivered from one irradiation angle.

Fig. 1d shows an example in which the first part is a first sub-arc R1" covering all of the patient's circumference and the second part is a second sub-arc R2" covering the same trajectory, that is a second revolution around the patient, in the same direction. As will be understood, the directions of R1" and R2" could also be opposite. In the case of a rotating patient support, it will be more feasible to continue the rotation in the same direction. If the gantry is rotating, it may be more feasible to reverse the rotation for the second sub-arc. The first and second sub-arcs R1", R2" will involve control points in a similar way to Fig. 1a or 1b. That is, the control points may define the same irradiation angles in both cases, but with different energy layers for each irradiation angle for each sub-arc, or may define different irradiation angles for the second sub-arc R2" than for the first sub-arc R1".

According to the present disclosure, an arc may include one or more sub-arcs, where the sub-arcs differ in direction, and/or the energy layers involved. According to an embodiment of the present disclosure, a first part of the delivery is in the form of a first sub-arc, having a first set of energy layers. A second part of the delivery involves at least a second part having a second set of energy layers that is different from the first set.

The second part may be in the form of the following:
- a second sub-arc having a different direction from the first sub-arc. The second sub-arc may follow the same trajectory as the first sub-arc but in a different direction, or run at an angle from the first sub-arc. One or both sub-arcs may also be non-linear, following any suitable non-linear trajectory.
- a second sub-arc having the same direction as the first sub-arc but a different set of energy layers. This is particularly suitable if the first sub-arc describes a full revolution around the patient, or if the first and second sub-arcs cover the same area of the patient in some other way. Combining higher energies with lower energies from the same irradiation direction is in many cases preferable for plan quality. However, if the different energies from the same or an adjacent direction are delivered consecutively, this can result in slow delivery times since the arc will contain many upward and downward energy switches from the same or adjacent directions. It is therefore often faster to make a full revolution with one set of energy layers and then deliver a second set of energy layers over a second revolution constituting a second sub-arc. This may also be suitable even if the first and/or second sub-arc only covers a portion of the circumference, which means that for part of the revolution the beam will be off.
- A static beam being delivered from one angle. The static beam is used to deliver more than one energy layer. This is particularly suitable where there are multiple energy layers for one direction, but a much lower number of energy layers for other directions. The directions requiring multiple energy layers could be chosen for delivery of static beams. This will result in a dynamic arc which stops at certain directions and delivers a larger number of energy layers and then continues.

In the first and second alternative above, the result may be seen as a multi-arc, comprising the first and second sub-arcs. In the third alternative, the result may be seen as a hybrid arc, comprising the first sub-arc and a static beam. The term "arc" in this document is intended to cover both multi-arcs and hybrid arcs according to this definition.

As will be understood, any combination of the above, in any order, is possible. For example, the treatment could consist in a static beam followed by a sub-arc, or vice versa. As another example, an arc may have several sub-arcs, with a static beam between all or some of them. These sub-arcs may have the same or opposite trajectories or different trajectories altogether. An arc may also comprise two or more sub-arcs describing the same full revolution with a static beam between.

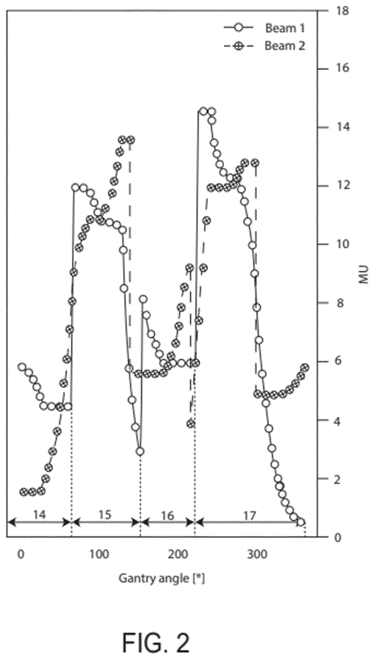

Fig. 2 illustrates, by way of example only, a possible way of arranging energy levels for the available irradiation directions within a first and a second sub-arc. In Fig. 2, the energy levels of the first sub-arc for each respective irradiation direction are marked as circles and the upward energy jumps of the first arc are indicated as solid lines. For the second sub-arc, the energy levels are marked as crossed circles and the upward energy jumps are indicated as dashed lines. In this example, it is assumed that the first arc is delivered in the direction from 0 degrees to 360 degrees and the second arc is delivered in the opposite direction. This is suitable, for example if a gantry delivers the first arc while in the first direction and then delivers the second sub-arc while returning to its initial position. As can be seen, the first sub-arc includes a number of changes to lower energy levels, but only three changes 11, 12, 13 to higher energy levels. Each uninterrupted sequence of changes to lower energy levels constitutes a sector. For the first sub-arc, the sectors 14, 15, 16, 17 are delimited by dotted lines. The number of changes to higher energy levels, signaling the start of a new sector, is kept low because such changes are more time-consuming than changing to a lower energy level. The second sub-arc similarly includes a number of changes to consecutively lower energy levels but only three jumps 21, 22, 23 to higher energy levels. In this way, the energy levels of the first and second sub-arcs can be selected to complement each other.

In the second option, the number of revolutions/changes of direction could be set by the user (e.g., 2 or 3) or be chosen by the algorithm based on goodness measures such as target coverage and delivery time. It could be combined with a maximum number of revolutions to have an upper limit, or with a combined goodness measure taking number of revolutions into account. The resulting arc could also include fractions of full revolutions, i.e., the resulting arc could have, for example, 2 1/3 revolutions, if this is beneficial.

In the third option, the directions for the static beams in the hybrid arc could be specified by the user or determined automatically by the algorithm based on goodness measures, which typically involve a combination of dosimetric measures and time measures.

Different methods for selecting static beam directions could include:
- Ray tracing in the patient in combination with goodness measures. A goodness measure could be based on the density variability in the path of the trace (in depth or laterally due to small perturbations). It could also be based on how clear the path to the target is with respect to OARs.
- Taking all static beams into account simultaneously to avoid selecting adjacent angles for static beams, since adjacent beams would usually not contribute to the same extent to robustness.
- Machine learning or deep learning methods
- Defining a number of candidate beam directions with candidate energy layers and running a first step of spot-weight optimization to determine which beam directions and layers that would have highest chances to meet the optimization objectives. For example, beam directions having many highly utilized energy layers can be selected, possibly taking the angles of the directions into account to avoid many close angles.

Static beams can be combined with several arc revolutions/rotation directions. The stops should then preferably be made to achieve the best delivery time, e.g.:
- Located at turning points of changing rotation direction, where there will already be a delay or a stop.
- Located before switching to an energy level, for which the energy switching time is longer than for other switches. As has been described previously, many machines suffer from longer switching times from lower to higher energies, and if the static beams can be placed at the location for upward switches, this can be beneficial from a delivery time perspective.

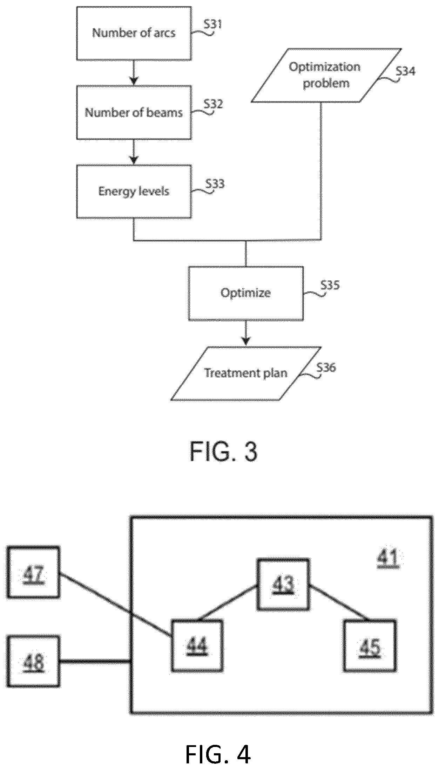

Fig. 3 illustrates different embodiments of the planning method according to the present invention. In a first, optional step S31, the number of sub-arcs to be included in the plan is pre-determined. Alternatively, a minimum and/or maximum value for the number of sub-arcs may be pre-determined, or the selection may be left to the optimization procedure.

In a second, optional step S32, the number of static beams to be included in the plan, or the minimum and/or maximum number of static beams, is pre-determined. The beam angles of the static beams may also be pre-determined. Alternatively, selection of numbers and/or beam angles may be left to the optimization procedure. In a third, optional step S33, the energy levels to be used in the sub-arc or sub-arcs, and in the static beams, are pre-selected. Methods of performing this pre-selection will be discussed below. The energy levels may also be left to the optimization procedure instead.

An optimization problem S34 is provided, which is arranged to optimize goodness measures such as target coverage and delivery time. In step S35 the optimization is performed based on the optimization problem. If the predetermining and pre-selection steps S31, S32, S33 have been performed, the results from these steps are also used as input to the optimization. The result of the optimization is a treatment plan defining an arc comprising one or more sub-arcs and optionally one or more static beams, in terms of control points including irradiation angles and energy levels.

Steps S31, S32, and S33 may be included or omitted independently of each other, and may be performed in any suitable order.

The multiple energy layers are selected by analyzing the target and the patient geometry for all revolutions simultaneously, or based on objectives such as dose or LET. This could be done by a pre-selection method or as part of the spot-weight optimization.

Pre-selection methods include, for example:
- Mirroring for every second sub-arc: create a solution where the first and the second sub-arc have upward energy jumps at the same locations. More generally speaking, the most costly energy level changes should be at the same locations; as will be understood this is typically the increases in energy level. As an example, the energy layers can first be selected for the first sub-arc and grouped into sectors with descending energy levels. When selecting the energy layers for the second sub-arc, the sectors are located between the same irradiation angles as for the first sub-arc. Alternatively, the selection of the location of the sectors could be made over the two sub-arcs simultaneously.
- Shifted Mirroring: for every two sub-arcs, the energy level changes are selected such that the most costly energy level changes, usually the upward jumps, for one sub-arc is located in the middle point between the most costly energy level changes of the other sub-arc.
- Optimized solution space: using a solution space where all sub-arcs use upward energy jumps at the same location, but where the energies between upward jumps are selected in such a way that the multiple sub-arcs improve the solution space in an optimal way. The optimization of the solution space could be based on:
   i. Maximizing covered target volume
   ii. Minimizing overlap of covered target volume
   iii. Maximizing different goodness measures as outlined above, influencing, e.g., delivery time or plan quality.
- Treating a multi-arc as a large single arc: where each sub-arc is added to the end of the previous sub-arc, creating a large single arc, and selecting the upward energy jumps for all sub-arcs without regard to the position of the jumps in other sub-arcs, but in such a way that the combined set of energy layers fulfil appropriate goodness measures with regard to target coverage and time in the same way as described above.

Selecting energy layers as part of the spot-weight optimization based on optimization objectives involves, for example:
- Energy layer reduction techniques, as disclosed, for example in US11027148B2 and EP3549636B1. The optimization starts with a larger number of energy layers than desired at the end of the optimization. The number of energy layers in use is then reduced during the optimization, for example by filtering, in which less utilized energy layers are removed, or by penalization techniques, in which the optimization includes a penalty on the number of utilized energy layers, or a combination of filtering end penalization. These energy layer reduction techniques are then applied to all the different parts simultaneously.
- Beam-splitting approach, as discussed in US 2021/0121150 A1. The optimization starts out with a limited number of static beams, and each beam is split into several sub-beams iteratively during the spot-weigh optimization. In this process, the energy layers from the original beam are redistributed over the new sub-beams until the sub-beams are placed close enough to form a continuous arc. In the current invention, the energy layers can be redistributed over at least two parts, one of which being a sub-arc.
- Other methods

As a final step after these methods for selecting energy layers, the total number of energy layers can be further reduced by the use of energy layer reduction techniques. This will reduce the delivery time, which is beneficial in the clinical workflow.

Fig. 4 is a schematic overview of a computer system in which the optimization according to the invention may be carried out. A computer 41 comprises a processor 43, a data memory 44 and a program memory 45. Preferably, one or more user input means 47, 48 is also present, in the form of a keyboard, a mouse, a joystick, voice recognition means and/or any other available user input means. The user input means may also be arranged to receive data from an external memory unit.

The program memory 45 holds a computer program arranged to control the processor to perform the optimization procedure according to the invention. Like the data memory 44, the program memory may also be implemented as one or several units as is seen fit. The data memory 44 holds input data that may be used in the treatment planning, and output data resulting from the planning. Input data includes patient data and information om clinical goals for the patient. Output data includes control point information such as energy levels, angles, spot weights and MU.

## Claims

1. A computer-implemented method of creating an ion-based radiotherapy treatment plan for treating a patient, the method including a spot-weight optimization procedure arranged to create the plan so that it involves an arc with a first part which is a first sub-arc, said sub-arc being a continuous sub-arc defined by a first set of control points defining a first set of energy levels distributed over a first trajectory involving different irradiation angles shifting the beam in a first direction, and so that the plan further involves a second part involving a second set of control points different from the first set of control points, the second set of control points defining a second set of energy levels, each of the first and second set including a plurality of energy levels, wherein
a. the second part is a static beam and the second set of control points define several energy levels from one irradiation angle, or
b. the second part is a second sub-arc and the second set of control points define a second trajectory along a second direction which is different from the first direction, or
c. the second part involves a second sub-arc moving in the first direction and the relative movement between the gantry and the patient over the arc describes more than a full revolution around the patient, or
d. the second part involves a second sub-arc at least partially overlapping the first sub-arc; and
wherein the method includes selecting the first and second sets of energy levels together.

2. A computer-implemented method according to claim 1, wherein the second direction of the second sub-arc is opposite from the first direction.

3. A computer-implemented method according to claim 1, wherein the second part is a second sub-arc and the second set of control points define a second trajectory along the first direction.

4. A computer-implemented method according to any one of the preceding claims, where second sub-arc and the first sub-arc at least partially overlap, and the irradiation angles are selected to complement each other.

5. A computer-implemented method according to any one of the preceding claims, where second sub-arc and the first sub-arc at least partially overlap, and at least some of the irradiation angles are selected to be the same for the first and the second sub-arcs.

6. A computer-implemented method according to any one of the preceding claims, wherein the energy layers of each part are grouped into at least a first and a second sector wherein the energy levels within each sector are arranged in order to reduce delivery time.

7. A computer-implemented method according to claim 5, wherein the energy layers are grouped into sectors in such a way that the energy levels decrease within each sector.

8. A computer-implemented method according to any one of the preceding claims, wherein the step of selecting the first and second sets of energy levels together includes a pre-selection step, wherein the energy levels are selected prior to the spot-weight optimization procedure.

9. A computer-implemented method according to claim 7, wherein the energy level sectors are the same for the first and second sub-arcs.

10. A computer-implemented method according to claim 7, wherein the pre-selection is performed by treating a multi-arc as a large single arc.

11. A computer-implemented method according to any of the claims 1 to 6, wherein the selection of energy levels is performed as part of a spot-weight optimization procedure based on dose-based optimization objectives.

12. A computer-implemented method according to any of the preceding claims, wherein the total number of energy levels is further reduced by applying an energy level reduction technique on the first and second set of energy levels together.

13. A computer program product comprising computer-readable code means which, when executed in a computer, will cause the computer to perform the method according to any one of the preceding claims.

14. A computer (41) having a processor (43) and a program memory (45), wherein the program memory (45) includes a computer program product according to claim 13, stored in such a way that it can be executed in the processor.

## Patentansprüche

1. Computerimplementiertes Verfahren zum Erstellen eines ionenbasierten Strahlentherapiebehandlungsplans zum Behandeln eines Patienten, wobei das Verfahren einen Punktgewichtsoptimierungsvorgang umfasst, der eingerichtet ist, um den Plan so zu erstellen, dass er einen Bogen mit einem ersten Teil beinhaltet, der ein erster Unterbogen ist, wobei der Unterbogen ein kontinuierlicher Unterbogen ist, der durch einen ersten Satz von Kontrollpunkten definiert ist, die einen ersten Satz von Energieniveaus definieren, die über eine erste Trajektorie verteilt sind, die unterschiedliche Bestrahlungswinkel beinhalten, die den Strahl in eine erste Richtung verschieben, und so, dass der Plan ferner einen zweiten Teil beinhaltet, der einen zweiten Satz von Kontrollpunkten beinhaltet, der sich von dem ersten Satz von Kontrollpunkten unterscheidet, wobei der zweite Satz von Kontrollpunkten einen zweiten Satz von Energieniveaus definiert, wobei jeder des ersten und des zweiten Satzes eine Vielzahl von Energieniveaus einschließt, wobei
a. der zweite Teil ein statischer Strahl ist und der zweite Satz von Kontrollpunkten mehrere Energieniveaus aus einem Bestrahlungswinkel definiert, oder
b. der zweite Teil ein zweiter Unterbogen ist und der zweite Satz von Kontrollpunkten eine zweite Trajektorie entlang einer zweiten Richtung definiert, die sich von der ersten Richtung unterscheidet, oder
c. der zweite Teil einen zweiten Teilbogen beinhaltet, der sich in die erste Richtung bewegt, und die relative Bewegung zwischen der Gantry und dem Patienten über den Bogen mehr als eine volle Umdrehung um den Patienten herum beschreibt, oder
d. der zweite Teil einen zweiten Unterbogen beinhaltet, der den ersten Unterbogen mindestens teilweise überlappt; und
wobei das Verfahren ein gemeinsames Auswählen des ersten und des zweiten Satzes von Energieniveaus einschließt.

2. Computerimplementiertes Verfahren nach Anspruch 1, wobei die zweite Richtung des zweiten Unterbogens der ersten Richtung entgegengesetzt ist.

3. Computerimplementiertes Verfahren nach Anspruch 1, wobei der zweite Teil ein zweiter Unterbogen ist und der zweite Satz von Kontrollpunkten eine zweite Trajektorie entlang der ersten Richtung definiert.

4. Computerimplementiertes Verfahren nach einem der vorstehenden Ansprüche, wobei sich der zweite Teilbogen und der erste Teilbogen mindestens teilweise überlappen und die Bestrahlungswinkel ausgewählt sind, um sich gegenseitig zu ergänzen.

5. Computerimplementiertes Verfahren nach einem der vorstehenden Ansprüche, wobei sich der zweite Teilbogen und der erste Teilbogen mindestens teilweise überlappen und mindestens einige der Bestrahlungswinkel für den ersten und den zweiten Teilbogen gleich ausgewählt werden.

6. Computerimplementiertes Verfahren nach einem der vorstehenden Ansprüche, wobei die Energieschichten jedes Teils in mindestens einen ersten und einen zweiten Sektor gruppiert sind, wobei die Energieniveaus innerhalb jedes Sektors eingerichtet sind, um eine Abgabezeit zu reduzieren.

7. Computerimplementiertes Verfahren nach Anspruch 5, wobei die Energieschichten auf eine solche Weise in Sektoren gruppiert sind, dass die Energieniveaus innerhalb jedes Sektors abnehmen.

8. Computerimplementiertes Verfahren nach einem der vorstehenden Ansprüche, wobei der Schritt des gemeinsamen Auswählens des ersten und des zweiten Satzes von Energieniveaus einen Vorauswahlschritt einschließt, wobei die Energieniveaus vor dem Punktgewichtsoptimierungsvorgang ausgewählt werden.

9. Computerimplementiertes Verfahren nach Anspruch 7, wobei die Energieniveausektoren für den ersten und den zweiten Unterbogen gleich sind.

10. Computerimplementiertes Verfahren nach Anspruch 7, wobei die Vorauswahl durch Behandeln eines Mehrfachbogens als ein großer Einzelbogen erfolgt.

11. Computerimplementiertes Verfahren nach einem der Ansprüche 1 bis 6, wobei die Auswahl der Energieniveaus als Teil eines Punktgewichtoptimierungsvorgangs basierend auf dosisbasierten Optimierungszielen durchgeführt wird.

12. Computerimplementiertes Verfahren nach einem der vorstehenden Ansprüche, wobei die Gesamtzahl der Energieniveaus durch Anwenden einer Technik für eine Reduzierung der Energieniveaus auf den ersten und den zweiten Satz von Energieniveaus zusammen weiter reduziert wird.

13. Computerprogrammprodukt, umfassend computerlesbare Codemittel, die, wenn sie auf einem Computer ausgeführt werden, den Computer veranlassen werden, das Verfahren nach einem der vorstehenden Ansprüche durchzuführen.

14. Computer (41), der einen Prozessor (43) und einen Programmspeicher (45) aufweist, wobei der Programmspeicher (45) ein Computerprogrammprodukt nach Anspruch 13 einschließt, das auf eine solche Weise gespeichert ist, dass es in dem Prozessor ausgeführt werden kann.

## Revendications

1. Procédé implémenté par ordinateur de création d'un plan de traitement de radiothérapie à base d'ions pour le traitement d'un patient, le procédé comportant une procédure d'optimisation de poids de point agencée pour créer le plan de sorte qu'il implique un arc avec une première partie qui est un premier sous-arc, ledit sous-arc étant un sous-arc continu défini par un premier ensemble de points de commande définissant un premier ensemble de niveaux d'énergie distribués sur une première trajectoire impliquant différents angles d'irradiation décalant le faisceau dans une première direction, et de sorte que le plan implique en outre une seconde partie impliquant un second ensemble de points de commande différent du premier ensemble de points de commande, le second ensemble de points de commande définissant un second ensemble de niveaux d'énergie, chacun du premier et du second ensemble comportant une pluralité de niveaux d'énergie, dans lequel
a. la seconde partie est un faisceau statique et le second ensemble de points de commande définit plusieurs niveaux d'énergie à partir d'un angle d'irradiation, ou
b. la seconde partie est un second sous-arc et le second ensemble de points de commande définit une seconde trajectoire le long d'une seconde direction qui est différente de la première direction, ou
c. la seconde partie implique un second sous-arc se déplaçant dans la première direction et le déplacement relatif entre le portique et le patient par-dessus l'arc décrit plus d'une révolution complète autour du patient, ou
d. la seconde partie implique un second sous-arc chevauchant au moins partiellement le premier sous-arc ; et
dans lequel le procédé comporte la sélection des premier et second ensembles de niveaux d'énergie conjointement.

2. Procédé implémenté par ordinateur selon la revendication 1, dans lequel la seconde direction du second sous-arc est opposée à la première direction.

3. Procédé implémenté par ordinateur selon la revendication 1, dans lequel la seconde partie est un second sous-arc et le second ensemble de points de commande définit une seconde trajectoire le long de la première direction.

4. Procédé implémenté par ordinateur selon l'une quelconque des revendications précédentes, où le second sous-arc et le premier sous-arc se chevauchent au moins partiellement, et les angles d'irradiation sont sélectionnés pour se compléter mutuellement.

5. Procédé implémenté par ordinateur selon l'une quelconque des revendications précédentes, où le second sous-arc et le premier sous-arc se chevauchent au moins partiellement, et au moins certains des angles d'irradiation sont sélectionnés pour être les mêmes pour les premier et second sous-arcs.

6. Procédé implémenté par ordinateur selon l'une quelconque des revendications précédentes, dans lequel les couches d'énergie de chaque partie sont groupées en au moins un premier et un second secteur dans lequel les niveaux d'énergie au sein de chaque secteur sont agencés afin de réduire le temps de délivrance.

7. Procédé implémenté par ordinateur selon la revendication 5, dans lequel les couches d'énergie sont groupées en secteurs d'une manière telle que les niveaux d'énergie diminuent au sein de chaque secteur.

8. Procédé implémenté par ordinateur selon l'une quelconque des revendications précédentes, dans lequel l'étape de sélection des premier et second ensembles de niveaux d'énergie conjointement comporte une étape de présélection, dans lequel les niveaux d'énergie sont sélectionnés avant la procédure d'optimisation de poids de point.

9. Procédé implémenté par ordinateur selon la revendication 7, dans lequel les secteurs de niveau d'énergie sont les mêmes pour les premier et second sous-arcs.

10. Procédé implémenté par ordinateur selon la revendication 7, dans lequel la présélection est mise en œuvre en traitant un multi-arc en tant que grand arc unique.

11. Procédé implémenté par ordinateur selon l'une quelconque des revendications 1 à 6, dans lequel la sélection de niveaux d'énergie est mise en œuvre dans le cadre d'une procédure d'optimisation de poids de point en fonction d'objectifs d'optimisation basés sur la dose.

12. Procédé implémenté par ordinateur selon l'une quelconque des revendications précédentes, dans lequel le nombre total de niveaux d'énergie est en outre réduit en appliquant une technique de réduction de niveau d'énergie sur les premier et second ensembles de niveaux d'énergie conjointement.

13. Produit programme informatique comprenant un moyen de code lisible par ordinateur qui, lorsqu'il est exécuté dans un ordinateur, amènera l'ordinateur à mettre en œuvre le procédé selon l'une quelconque des revendications précédentes.

14. Ordinateur (41) ayant un processeur (43) et une mémoire de programme (45), dans lequel la mémoire de programme (45) comporte un produit programme informatique selon la revendication 13, stocké d'une manière telle qu'il peut être exécuté dans le processeur.
